# EUROPEAN PATENT SPECIFICATION

(11) **EP 3 150 109 B1**
(45) Date of publication and mention of the grant of the patent: **05.07.2023**
(21) Application number: 16191601.0
(22) Date of filing: 29.09.2016
(51) Int. Cl.: A61B 3/10

(54) **FUNDUS IMAGING DEVICE**
AUGENHINTERGRUNDABBILDUNGSVORRICHTUNG
DISPOSITIF D'IMAGERIE DU FOND DE L'OEIL

(30) Priority: 30.09.2015 JP 2015195448; 30.09.2015 JP 2015195449
(43) Date of publication of application: 05.04.2017
(73) Proprietor: Nidek Co., Ltd., Gamagori Aichi (JP)
(72) Inventor: SASAKI, Joji, Gamagori, Aichi (JP); HANEBUCHI, Masaaki, Gamagori, Aichi (JP)
(74) Representative: Hoefer & Partner Patentanwälte mbB

(56) References cited:
- US-A- 5 815 242
- US-A1- 2007 030 449
- US-A1- 2010 141 895
- US-A1- 2010 150 415
- US-A1- 2013 093 996
- US-A1- 2014 327 882
- US-A1- 2015 216 408
- Anonymous: "Quadric", Wikipedia, 29 July 2015 (2015-07-29), XP055782040, Retrieved from the Internet: URL:https://en.wikipedia.org/w/index.php?t itle=Quadric&oldid=673677934 [retrieved on 2021-03-04]

## Description

### BACKGROUND

The present invention relates to a fundus imaging device.

The related art discloses a fundus imaging device that obtains an image of a fundus by scanning the fundus of a subject's eye with light via an optical scanner. For example, a scanning laser ophthalmoscope (SLO) obtains a front image of a fundus as the result of scanning the fundus with light.

The capturing of a wide-range image of a fundus via such a fundus imaging device is tried (for example, refer to JP-A-2014-138904).

US 2015/0216408 A1 describes a scanning laser ophthalmoscope for imaging the retina of an eye according to the preamble of claim 1 comprising a source of collimated light, a scanning device, a scan transfer device and a detector. The scan transfer device has a first focus at which an apparent point source is provided and a second focus at which an eye may be accommodated. The scan transfer device transfers a two-dimensional collimated light scan from the apparent point source into the eye. An optical coherence tomography system is combined with the SLO, the OCT system providing OCT reference and sample beams. The OCT sample beam propagates along the same optical path as of the SLO collimated light through the scan transfer device. An aberration compensator automatically compensates for systematic aberrations and/or changes in wavefront introduced by scanning elements and the scan transfer device as a function of scan angle.

US 2013/0093996 A1 describes a scanning ophthalmoscope for scanning the retina of an eye and a method of scanning the retina of an eye. The ophthalmoscope comprises a source of collimated light, a first scanning element, a second scanning element and a scan relay device having two foci, The source of collimated light, the first and second scanning elements and the scan relay device combine to provide a two-dimensional collimated light scan from an apparent point source. The scanning ophthalmoscope further comprises a scan transfer device, wherein the scan transfer device has two foci and at least one vertex and the apparent point source is provided at a first focus of the scan transfer device and an eye is accommodated at a second focus of the scan transfer device, and wherein the scan transfer device transfers the two-dimensional collimated light scan from the apparent point source into the eye, two foci of the scan relay device and the two foci of the scan transfer device define a first plane and the two foci and the at least one vertex of the scan transfer device define a second plane and wherein the first and second planes are substantially parallel.

US 2010/0141895 A1 describes a scanning opthalmoscope provided for scanning the retina of an eye and comprising a source of collimated light, a first scanning element, a second scanning element, scan compensation means, wherein the source of collimated light, the first and second scanning elements and the scan compensation means combine to provide a two-dimensional collimated light scan from an apparent point source, and the scanning opthalmoscope further comprises scan transfer means, wherein the scan transfer means has two foci and the apparent point source is provided at a first focus of the scan transfer means and an eye is accommodated at a second focus of the scan transfer means.

US 2014/0327882 A1 describes a scanning laser ophthalmoscope for scanning the retina of an eye comprising a light source emitting a beam of light, scan relay elements, wherein the light source and the scan relay elements provide a two-dimensional scan of the light beam which is transferred from an apparent point source at a pupillary point of the eye to the retina of the eye, and a static aberration correction element which has a shape defined to provide correction of aberrations of at least some of the scan relay elements and a location within the ophthalmoscope chosen to provide correction of aberrations of at least some of the scan relay elements, which location maintains transfer of the beam of light from the apparent point source at the pupillary point of the eye to the retina of the eye.

US 2007/0030449 A1 describes an apparatus for obtaining an image of the eye, which has a light source for providing an incident illumination and an apertured mirror for directing at least a portion of the incident illumination along an optical axis. A curved objective mirror directs the incident illumination received along the optical axis toward the retina of the eye and directs image-bearing light reflected from the retina back along the optical axis. The apertured mirror transmits the image-bearing light reflected from the retina toward a sensor for obtaining an image of the retina thereby.

There is room for improvement in an optical system of a device that captures a wide-range image of a fundus via scanning. For example, a difference in aberration between scan positions is likely to affect an image. It is considered that as disclosed in JP-A-2014-138904, if an objective optical system for widening the angle of a scanning range is formed of a lens system, the reflection of light from the surface of a lens may affect an image.

This disclosure is made in light of at least one of the problems in the related art, and a technical object of this disclosure is to provide a fundus imaging device with a new optical system which is capable of capturing a good wide-range image of a fundus.

This object is solved by the features of the independent claim. The dependent claims contain advantageous embodiments of the present invention.

### BRIEF DESCRIPTION OF THE DRAWINGS

Fig. 1 is a view illustrating a schematic configuration of optical systems of a fundus imaging device of an embodiment.
Fig. 2 is a view illustrating an objective optical system of a first example.
Fig. 3 is a block diagram illustrating an electrical configuration of a fundus imaging device of the first example.
Fig. 4 is a view illustrating an objective optical system of a second example.
Fig. 5 is a view illustrating an objective optical system of a third example.

### DESCRIPTION OF ILLUSTRATIVE EMBODIMENTS

Hereinafter, a typical embodiment of this disclosure will be described with reference to the accompanying drawings.

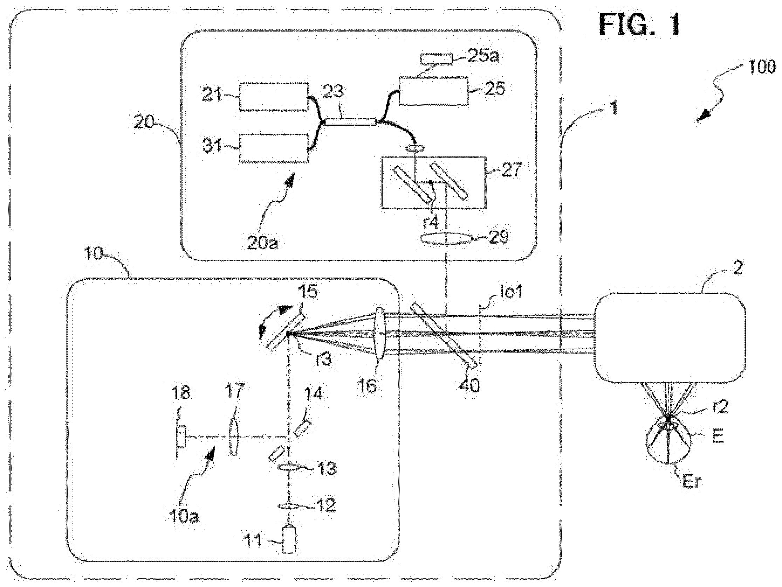

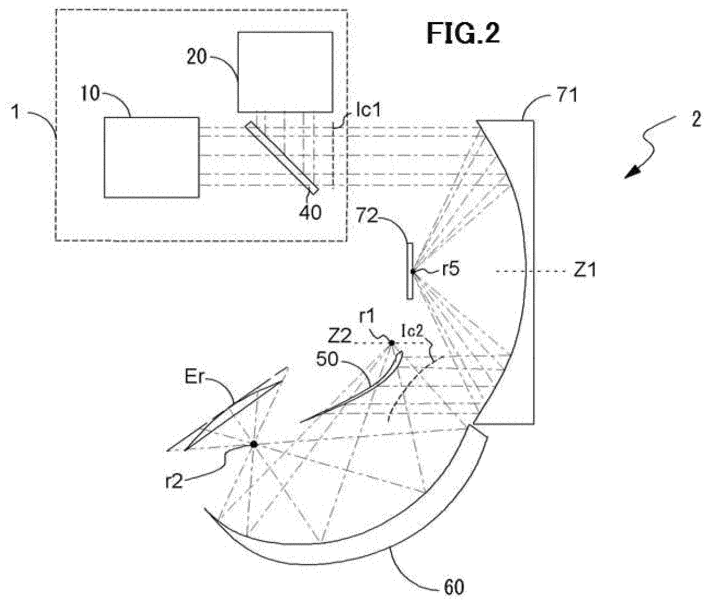

First, an overview of a fundus imaging device (hereinafter, is abbreviated as an "imaging device") of this disclosure will be described with reference to Figs. 1 and 2. An imaging device 100 obtains an image of a fundus by scanning the fundus of a subject's eye E with light. An image of a fundus may be a front image or may be a tomographic image. The scanning range of the imaging device 100 of this disclosure when scanning a fundus with light may be a wide range. For example, a full angle range of 100° or greater may be scanned with light, and an image of this scanning range may be obtained.

As illustrated in Fig. 1, the imaging device 100 includes a scanning optical system 1 and an objective optical system 2. The imaging device 100 includes light receiving optical systems 10a and 20a. The scanning optical system 1 includes optical scanners 15 and 27 which change the travelling direction of light so as to scan a fundus Er with light from a light source. The objective optical system 2 is disposed between the scanning optical system 1 (more specifically, the optical scanners 15 and 27) and the subject's eye E. The objective optical system 2 is used to guide light from the optical scanners 15 and 27 to the fundus Er. The light guided to the fundus Er by the objective optical system 2 is reflected and scattered by the fundus Er, and thus is emitted from a pupil. The light emitted from the pupil is received by respective detectors 18 and 31 of the light receiving optical systems 10a and 20a. As a result, images of the fundus are formed based on received light signals output from the detectors 18 and 31. For example, the detectors 18 and 31 may be provided in the objective optical system 2.

The scanning optical system 1 may include one of an SLO optical system 10 and an OCT optical system 20. As illustrated in Fig. 1, the scanning optical system 1 may include both. The SLO optical system 10 includes the optical scanner 15, and is used to obtain a front image of the fundus Er. The SLO optical system 10 may be a line-scan SLO optical system that scans the fundus Er with line-shaped light fluxes in at least one direction via the optical scanner 15, or may be a point-scan SLO optical system that two-dimensionally scans the fundus Er with dot-shaped light fluxes via the optical scanner 15. The OCT optical system 20 includes the optical scanner 27, and non-invasively obtains a tomographic image of the fundus Er via optical interference by scanning the fundus Er with measurement light via the optical scanner 27. In the embodiment, the optical scanners 15 and 27 and an anterior ocular segment of the subject's eye E have a conjugate relationship in position with respect to the objective optical system 2. In this disclosure, "conjugation" is not necessarily limited to a perfectly conjugate relationship. In this disclosure, in addition to representing a perfectly conjugate relationship, a "conjugate" relationship may represent a positional relationship deviating from the perfectly conjugate relationship in an allowable accuracy range.

As illustrated in Fig. 1, in the configuration in which the scanning optical system 1 includes both the SLO optical system 10 and the OCT optical system 20, the scanning optical system 1 may include an optical path coupling member 40 that couples optical paths of the SLO optical system 10 and optical paths of the OCT optical system 20 together. The wavelength of light emitted from a light source 11 of the SLO optical system 10 is preferably different from the wavelength of light emitted from a light source 21 of the OCT optical system 20. A dichroic mirror which wavelength-selectively couples optical paths together is preferably used as the optical path coupling member 40. The optical path coupling member 40 is not necessarily limited to a dichroic mirror, and may be other optical path coupling members such as a half mirror. In Fig. 1, the optical path coupling member 40 couples the respective optical paths of the SLO optical system 10 and the OCT optical system 20 together between the optical scanners of the optical systems and the objective optical system 2. As such, in the embodiment, the optical scanners are respectively provided in the SLO optical system 10 and the OCT optical system 20. As a result, it is possible to easily obtain a tomographic image at an arbitrary position in parallel with acquiring a front image.

The light receiving optical system 10a receives fundus reflected light of irradiated light from the SLO optical system 10 via a detector 18. Fundus reflected light of irradiated light (measurement light) from the OCT optical system 20 is combined with reference light, and then is received by the detector 31 of the light receiving optical system 20a.

The objective optical system 2 forms a pivot point (second pivot point in the embodiment) in the anterior ocular segment (for example, pupillary zone) of the subject's eye E around which light through the optical scanners 15 and 27 turns in correspondence with the operation of the optical scanners 15 and 27. As illustrated in Fig. 2, the objective optical system 2 may be a mirror system that does not include lens elements. In this case, it is possible to prevent an image of the fundus from being affected by noise (for example, causing flare in a front image) caused by the objective optical system 2.

The objective optical system 2 includes at least a first mirror 50 and a second mirror 60. As illustrated in Fig. 2 in addition to the first mirror 50 and the second mirror 60, the objective optical system 2 of the embodiment may include optical members such as a mirror.

In the embodiment, the first mirror 50 is disposed between the scanning optical system 1 and the second mirror 60. Light from the optical scanners 15 and 27 is incident to the first mirror 50, and the first mirror 50 relays the incident light to the second mirror 60.

In the embodiment, the first mirror 50 reflects light from the optical scanners 15 and 27, and thus forms a first pivot point (represented by reference sign r1 in Fig. 2) around which light (specifically, light reflected by the first mirror) turns in correspondence with the operation of the optical scanners 15 and 27.

In the embodiment, the first mirror 50 is a mirror having a focal point, and has positive power or negative power. The first mirror 50 forms the first pivot point at the focal point of the first mirror 50.

The first mirror 50 preferably is an aspherical mirror, the mirror surface of which is a quadric surface. A quadric surface is formed along a trajectory that is obtained by rotating a quadratic curve (also referred to as a conic section) around a symmetrical axis. An aspherical mirror as the first mirror 50 having a quadric surface may be at least any one of a paraboloidal mirror, a hyperboloidal mirror, and an ellipsoidal mirror. The first mirror 50 may has a mirror surface other than a quadric surface of an aspherical mirror. For example, the first mirror 50 may be a spherical mirror, or may have a shape other than a spherical surface. The first mirror 50 of the embodiment is formed of one mirror. Alternatively, the first mirror 50 formed of one mirror may be replaced with a combination of multiple mirrors.

The second mirror 60 has positive power. The second mirror 60 further reflects the light reflected by the first mirror 50, and thus forms the second pivot point around which light to be emitted from the objective optical system 2 to the subject's eye E turns.

In the embodiment, the subject's eye E is disposed at a focal point r2 of two focal points r1 and r2 of the second mirror 60. More specifically, the anterior ocular segment (for example, the position of the pupil) is disposed at the position of the focal point r2. The first mirror 50 and the second mirror 60 are disposed such that the first pivot point formed by the first mirror 50 coincides with a focal point r1 of the second mirror 60. In the embodiment, as a result, the second pivot point r2, around which light traveling from the objective optical system 2 toward the fundus Er turns, is formed at the focal point r2.

The second mirror 60 illustrated in Fig. 2 is a spheroidal mirror (ellipsoidal mirror). In this case, light, which passes through the focal point r1 and is reflected by the mirror surface of the spheroidal mirror, always passes through the focal point r2. Accordingly, light with which the subject's eye E is irradiated is disposed at the focal point r2 is unlikely to causing vignetting in the pupil. Since the occurrence of vignetting is prevented, it is possible to capture a wide angle image of the fundus.

A curved mirror other than a spheroidal mirror may be adopted as the second mirror 60. The curved mirror may be an aspherical mirror (as a specific example, a paraboloidal mirror, a pair of hyperboloidal mirrors, or the like) having a quadric mirror surface similar to a spherical mirror. The curved mirror may be an aspherical mirror having a shape other than a quadric mirror surface. The second mirror 60 of the embodiment is formed of one mirror. Alternatively, the second mirror 60 formed of one mirror may be replaced with a combination of multiple mirrors. The shape and the size of the second mirror 60 are suitably set according to the angle of a captured image. For example, in the embodiment, the second mirror 60 may have such a shape and a size that it is possible to capture an image of a full angle of approximately 120°.

Due to the configuration of the mirrors, the objective optical system 2 of the embodiment further decreases the swing angle of light which is incident to the first mirror 50 through the optical scanners 15 and 27, compared to a swing angle with respect to the second pivot point r2.

In the embodiment, one or both of the first mirror 50 and the second mirror 60 cause the angle of an imaging range to be widened to a desired image angle. As in the example illustrated in Fig. 2, at least the first mirror 50 further increases the swing angle of light travelling from the first pivot point r1 toward the second mirror 60 compared to the swing angle of light incident to the first mirror 50 through the optical scanners 15 and 27 (more specifically, the swing angle of light incident to the objective optical system 2). As a result, an image angle may be widened. It is possible to obtain the shape and the disposition of the second mirror 60 via simulation or the like using a beam tracing method.

If the first mirror 50 is not provided, and one of the respective optical scanners 15 and 27 of the SLO optical system 10 and the OCT optical system 20 is disposed at the focal point r1 of the second mirror 60, and light is directly guided from the optical scanner to the second mirror 60, the following problem occurs. That is, it is difficult to ensure a sufficient space between the optical scanner and the second mirror 60. It is necessary to increase the swing angle of the optical scanner so as to scan a wide range on the fundus with light. In this case, it is more difficult to suitably dispose the optical path coupling member 40 in the space between the optical scanner and the second mirror 60. The optical path coupling member 40 may have incidence angle dependence. For example, a dichroic mirror which is an example of the optical path coupling member 40 may not be able to suitably transmit or reflect light that is incident thereto at an incidence angle which significantly deviates from a design value. For this reason, even if it is possible to dispose an optical path coupling member such as a dichroic mirror between the second mirror 60 and the optical scanner disposed at the position of the focal point r1, in this case, image quality at the image angle (wide angle side) of a portion of a fundus image deteriorates. For this reason, the capturing of a wide-range image of the fundus by one or both of the SLO optical system and the OCT optical system becomes difficult.

In contrast, in the embodiment, the first mirror 50 is interposed between the optical scanners 15 and 27 and the second mirror 60. As the result of providing the first mirror 50, the objective optical system 2 causes light to turn around the first pivot point r1 at a larger swing angle compared to the swing angle of light that is incident to the objective optical system 2 from the scanning optical system 1. In other words, it is possible to further decrease the swing angle of light which is incident to the first mirror 50 through the optical scanners 15 and 27, compared to the swing angle with respect to the first pivot point r1. In other words, it is possible to form an area, in which the swing angle of light is smaller than the swing angle light incident to the first mirror 50, at a position closer to a light source side than to the first mirror 50. As a result, it is possible to easily ensure a space, in which the optical path coupling member 40 is disposed, in the area in which the swing angle of light is smaller than the swing angle of light incident to the first mirror 50. It is possible to reduce a problem of incidence angle dependence of the optical path coupling member 40 by disposing the optical path coupling member 40 in this area. For example, as illustrated in Fig. 1, it is possible to satisfactorily couple the optical paths of the SLO optical system 10 and the optical paths of the OCT optical system 20 together by disposing the optical path coupling member 40 between the optical scanner 15 of the SLO optical system 10 and the first mirror 50 and between the optical scanner 27 of the OCT optical system 20 and the first mirror 50. As a result, it is possible to acquire a good front image and a good tomographic image.

As illustrated in Fig. 1, the SLO optical system 10 and the OCT optical system 20 may be telecentric with respect to an object side. In this case, since the incidence angle of light incident to the optical path coupling member 40 is the same regardless of scan position, it is possible to satisfactorily prevent a loss in the amount of light caused by the coupling of the optical paths, and to obtain a good image of the fundus.

As illustrated in Fig. 2, in a case where a spheroidal mirror is used as the second mirror 60, the asymmetric distortion (for example, trapezoidal distortion) of an image plane occurs due to the second mirror 60. It is considered that even if the second mirror 60 adopts a shape other than that of a spheroidal mirror, the distortion of an image plane may occur.

The distortion of the image plane caused by the second mirror 60 is corrected by the first mirror 50. The first mirror 50 may be disposed inclined with respect to the second mirror 60 such that the distortion of the image plane is be corrected. The inclination referred to here implies that the first mirror 50 is disposed in such a way that the axis (for example, the symmetrical axis of a quadratic curve of a mirror surface of an aspherical mirror) is inclined with respect to a beam (mainly, beam passing through the optic axis of the subject's eye E) passing through the center of optical paths between the first mirror 50 and the second mirror 60. The amount of inclination is desirably set to some extent that at least asymmetric components of the distortion of the image plane are cancelled out. That is, the amount of inclination may be adopted such that the remaining distortion of the image plane is axis-symmetric.

In the example illustrated in Fig. 2, it is considered that as the result of using a spheroidal mirror as the second mirror 60, the image plane of an intermediate image Ic2 caused by fundus reflected light is obliquely inclined with respect to the optical axis of the optical system. It is considered that even if the second mirror 60 adopts a shape other than that of a spheroidal mirror, the inclination of an image plane may occur.

In contrast, the imaging device 100 of the embodiment may be provided with an optical member that corrects the inclination of an image plane which occurs due to fundus reflected light being reflected by the first mirror 50 and the second mirror 60.

For example, an optical member which corrects the inclination of an image plane may be disposed on common optical paths of projected light optical paths along which light from the light sources 11 and 21 is guided to the subject's eye E, and light receiving optical paths along which fundus reflected light is guided to the detectors 18 and 31. Optical members may be respectively disposed on independent optical paths of the projected light optical paths and on independent optical paths of the light receiving optical paths is reduced.

As a specific example in which an optical member is disposed on common optical paths, the objective optical system 2 may be provided with correction mirror systems 71 and 72 which are optical members to correct the inclination of an image plane caused by fundus reflected light. The correction mirror systems 71 and 72 further incline an image plane so as to cancel out the inclination of the image plane caused by the first mirror 50 and the second mirror 60. As a result, as illustrated in Fig. 2, the inclination of the image plane of an intermediate image (for example, intermediate image Ic1 in Fig. 2) formed by the fundus reflected light through the correction mirror systems 71 and 72 is reduced.

As another specific example, a lens inclined with respect to the optical axis thereof may be disposed on the common optical paths such that the inclination of an image plane is corrected. For example, in the example illustrated in Fig. 1, scan lenses 16 and 29 may be inclinedly disposed.

In a case where optical members are respectively disposed on the independent optical paths of the projected light optical paths and on the independent optical paths of the light receiving optical paths, at least the detector 18 of the SLO optical system 10 may be used as one of the optical members. For example, in a case where the SLO optical system 10 is a line-scan SLO optical system, a line sensor or an area sensor is used as the detector 18. In this case, it is possible to correct the inclination of an image plane by inclining the detector 18 with respect to an optical axis of the light receiving optical system 10a. The amount of inclination of the detector 18 is desirably adjusted such that a Scheimpflug relationship is established between the fundus Er and the objective optical system 2. Furthermore, independent optical paths of the projected light optical system 10a of the SLO optical system 10 may include an optical member such as a lens, which is disposed inclined with respect to the optical axis thereof.

In a case where the SLO optical system 10 is a point-scan SLO optical system, an optical member (an inclinedly disposed lens, a correction mirror system, or the like) separate from the detector is desirably provided on the light receiving optical paths from the detector to the subject's eye E. Accordingly, it is possible to prevent the confocal position of the fundus Er from moving in correspondence with a change in the scan position of the optical scanner 15 by preventing the inclination of an image plane. As a result, it is possible to form a stationary conjugate point of the fundus between the optical member and the detector 18. For this reason, it is possible to dispose an aperture for removing undesired light at a stationary confocal position, and to obtain a good front image.

### <Example>

The imaging device 100 of a first example will be described with reference to Fig. 1. The imaging device 100 of the first example includes the scanning optical system 1 illustrated in Fig. 1 and the objective optical system 2 illustrated in Fig. 2. The scanning optical system 1 includes the SLO optical system 10; the OCT optical system 20; and a dichroic mirror (optical path coupling member in the first example) 40.

### <SLO Optical System>

First, the SLO optical system 10 will be described. The SLO optical system 10 includes mainly the optical scanner 15 that two-dimensionally scans the fundus with light (illumination light) emitted from a light source; the detector 18; and the light receiving optical system 10a that receives fundus reflected light (of illumination light), which passes through a confocal point of the fundus Er, via the detector. In the first example, a line-scan type SLO optical system is used as the SLO optical system 10, and a line sensor is used as the detector 18. In this case, in addition to the optical scanner 15 and the detector 18, the SLO optical system 10 may include the light source 11; a collimating lens 12; a columnar lens 13; an aperture mirror 14; the scan lens 16; and a condenser lens 17. The light receiving optical system 10a of the SLO optical system 10 of the first example is formed of the optical scanners 15, the scan lens 16, the condenser lens 17, and the line sensor (detector) 18 among these elements.

In the first example, the light source 11 emits light (for example, laser beams) having infrared wavelengths. An LED light source, an SLD light source, or the like may be used as the light source 11. Light from the light source 11 is collimated by the collimating lens 12, and then is concentrated by the columnar lens 13. Thereafter, the light passes through an aperture of the aperture mirror 14, and is guided to the optical scanner 15. Light emitted from the light source 11 is not necessarily limited to infrared light. For example, light from the light source 11 may be white light, or may be combined light which is a combination of two or more colors (for example, red, blue, and green) of light.

In the first example, a galvanometer mirror may be used as the optical scanner 15. The optical scanner 15 is not necessarily limited to a galvanometer mirror. A galvanometer mirror may be replaced with any one of other optical scanners (a resonant mirror, a polygon mirror, and the like) which operates a reflective mirror, an acousto-optical device, and the like. The optical scanner 15 is disposed at a conjugate position with respect to the pupil of the subject's eye E.

The scan lens 16 turns light through the optical scanner 15 into beams (that is, telecentric beams) parallel to an optical axis L1 of the scanning optical system 1. That is, in the first example, the scan lens 16 is disposed such that a focal point of the scan lens 16 coincides with that of the optical scanner 15 (pivot point r3 of the optical scanner 15). Accordingly, the SLO optical system 10 of the first example is telecentric with respect to an object side. Light passing through the scan lens 16 further passes through the dichroic mirror 40, and is incident to the objective optical system 2. In the first example, the dichroic mirror 40 has spectral characteristics by which the dichroic mirror 40 transmits light from the SLO optical system 10, and reflects light from the OCT optical system 20. To be telecentric with respect to an object side implies a state of being telecentric with respect to a subject's eye E side when beams viewed from a light source 11 or 21 side.

Light from the SLO optical system 10 is guided to the fundus Er by the objective optical system 2, and then is scattered and reflected by the fundus Er. As a result, fundus reflected light is emitted from the pupil, and travels along optical paths opposite to those when light is projected. The fundus reflected light is emitted from the objective optical system 2 toward the scanning optical system 1, passes through the dichroic mirror 40, and is incident to the light receiving optical system 10a of the SLO optical system 10. In the light receiving optical system 10a, the fundus reflected light passes through the scan lens 16, is reflected by the optical scanner 15, and travels toward the aperture mirror 14. Thereafter, the fundus reflected light reflected by the aperture mirror 14 is concentrated by the condenser lens 17, and is received by the detector 18. In the embodiment, a front image of the fundus is formed based on signals which are output from the detector 18 based on one frame of light scanning performed by the optical scanner 15.

### <OCT Optical System>

Hereinafter, the OCT optical system 20 will be described. The OCT optical system 20 may include the light source 21; an optical splitter (coupler in the example illustrated in Fig. 1) 23; the optical scanner 27; and the detector 31. The OCT optical system 20 may further include the scan lens 29 and a reference optical system 25.

Fourier domain types such as a swept source-OCT (SS-OCT) type and a spectral domain-OCT (SD-OCT) type may be used as the OCT optical system 20. Hereinafter, as an example, a swept source-OCT (SS-OCT) type used as the OCT optical system 20 will be described.

The light source 21 is a variable wavelength light source (wavelength scanning light source) that changes an emitted wavelength at a high speed. The light source 21 changes the wavelength of emitted light. The detector 31 may be a balanced detector formed of light receiving elements.

The OCT optical system 20 splits light emitted from the light source 21 into measurement light and referent light via the coupler (splitter) 23.

The OCT optical system 20 guides the measurement light to the objective optical system 2 via the optical scanner 27. The OCT optical system 20 guides the reference light to the reference optical system 25. The optical scanner 27 scans the fundus Er with the measurement light in an XY direction (traverse direction). The optical scanner 27 is formed of two galvanometer mirrors. The angle of reflection may be arbitrarily adjusted by a drive mechanism (not illustrated). Other optical scanners (a resonant mirror, a polygon mirror, and the like) which operates a reflective mirror, an acousto-optical device, and the like may be used instead of galvanometer mirrors.

The scan lens 29 turns light through the optical scanner 27 into beams (that is, telecentric beams) parallel to the optical axis of the scanning optical system 1. That is, in the first example, the scan lens 29 is disposed such that a focal point of the scan lens 29 coincides with that of the optical scanner 27 (for example, intermediate point r4 between an X-scan optical scanner and a Y-scan optical scanner). Accordingly, the OCT optical system 20 of the embodiment is telecentric with respect to an object side. Light passing through the scan lens 29 is reflected by the dichroic mirror 40, and then is incident to the objective optical system 2.

Similar to light from the SLO optical system 10, the light from the OCT optical system 20 is guided to the fundus Er by the objective optical system 2, and then is scattered and reflected by the fundus. As a result, fundus reflected light of the measurement light travels through the objective optical system 2 along optical paths opposite to those when light is projected, and is emitted toward the scanning optical system 1. The fundus reflected light of the measurement light is reflected by the dichroic mirror 40, and is incident to a detective optical system 20a of the OCT optical system 20 (light receiving optical system of the OCT optical systems 20). That is, the fundus reflected light passes through the scan lens 29, and is incident to the coupler (splitter) 23 through the optical scanner 27. Thereafter, the reflected light of the measurement light is combined with reference light by the optical splitter (coupler) 23, and interferes therewith.

The reference optical system 25 generates reference light that is to be combined with reflected light acquired via reflection of measurement light by the fundus Er. The reference optical system 25 may be a Michelson type, or may be a Mach-Zehnder type. In Fig. 1, the reference optical system 25 includes a reflective optical system (for example, a reference mirror), and guides reference light to the detector 31 by reflecting light from the coupler 23 via the reflective optical system. As another example, the reference optical system 25 may include a transmissive optical system (for example, an optical fiber), and may guide light from the coupler 23 to the detector 31 by transmitting the light instead of returning the light.

The imaging device 100 moves at least a portion of the optical members disposed in the OCT optical system 20 in an optical axial direction so as to adjust an optical path length difference between measurement light and reference light. For example, the reference optical system 25 includes a configuration element that adjusts an optical path length difference between measurement light and reference light by moving an optical member (for example, a reference mirror not illustrated) on reference optical paths. The reference mirror is moved in the optical axial direction by driving a drive mechanism 25a. A configuration element for changing an optical path length difference may be disposed on optical paths of measurement light. That is, the optical path length difference between measurement light and reference light may be adjusted by changing the optical path length of measurement light.

The detector 31 receives interference signal light which is a combination of measurement light and reference light. The detector 31 detects interference signal light. If an emitted wavelength from the light source 21 is changed, the detector 31 receives interference signal light in correspondence with the change, and as a result, receives the interference signal light as spectral interference signal light. Depth profiles (may be referred to as A scan or OCT data) at one point on the fundus are formed based on spectral interference signals output from the detector 31. The depth profiles are a reflection intensity distribution of measurement light in a depth direction of the fundus. Two-dimension OCT data (a tomographic image of the fundus, OCT angiography, or the like) is formed by aligning the depth profiles (OCT data).

### <Objective Optical System>

Hereinafter, the objective optical system 2 of the first example will be described with reference to Fig. 2. In the example illustrated in Fig. 2, the second mirror 60 is one spheroidal mirror. The second mirror 60 has the two focal points r1 and r2. The subject's eye E is disposed at the focal point r2 of these.

In the first example, the first mirror 50 is a paraboloidal mirror. In the first example, the first mirror 50 and the second mirror 60 are disposed such that the focal point of the first mirror 50 which is a paraboloidal mirror coincides with one (the focal point r1) of the two focal points of the second mirror 60 which is a spheroidal mirror. The remaining one (the focal point r2) of the two focal points of the second mirror 60 is disposed on the anterior ocular segment of the subject's eye E. In this case, a convex surface of the first mirror 50 and a concave surface of the second mirror 60 are used as reflective surfaces. The paraboloidal mirror converts light, which is incident thereto while being parallel to a symmetrical axis z2, into light that is emitted from the focal point r1 by appearance. The first mirror 50 concentrates the light, which is emitted from the focal point r1, to the focal point r2. For this reason, in the first example, light from the SLO optical system 10 and the OCT optical system 20 which are telecentric with respect to the object side is incident to the first mirror 50 while being parallel to the symmetrical axis of the first mirror 50. Therefore, light from the SLO optical system 10 and light from the OCT optical system 20 are capable of turning around the focal point r2 that is a pivot point positioned on the anterior ocular segment of the subject's eye E. Since the first mirror 50 and the second mirror 60 of the objective optical system 2 have the aforementioned shapes and are disposed as described above, it is possible to decrease the swing angle of light incident to the first mirror 50 through the optical scanners 15 and 27, and to scan a wide range on the fundus with light.

The first mirror 50 formed of a paraboloidal mirror has been described, and is one example of specific examples of an aspherical mirror that widens an imaging angle. An aspherical mirror other than a paraboloidal mirror may be adopted as the first mirror.

In the first example, in addition to the first mirror 50 and the second mirror 60, the correction mirror systems 71 and 72 are provided in the objective optical system 2. In the first example, the correction mirror systems 71 and 72 are respectively formed of a paraboloidal mirror and a plane mirror, and are also referred to as a paraboloidal mirror 71 and a plane mirror 72. The correction mirror systems 71 and 72 correct the inclination of an image plane due to fundus reflected light being reflected by the first mirror 50 which is a paraboloidal mirror and the second mirror 60 which is a spheroidal mirror. In the first example, light from the SLO optical system 10 and the OCT optical system 20 which are telecentric with respect to the object side is incident to the first mirror 50 while being parallel to the symmetrical axis z2.

In the first example, the paraboloidal mirror 71 has a concave surface that is formed symmetrical with respect to a symmetrical axis z1. Light from the scanning optical system 1 is incident to the correction mirror system 71 while being parallel to the symmetrical axis z1. The plane mirror 72 is disposed at the position of a focal point r5 of the paraboloidal mirror 71 while confronting the paraboloidal mirror 71. If light from the scanning optical system 1 is incident to the correction mirror systems 71 and 72, the light is reflected by the paraboloidal mirror 71, the plane mirror 72 (that is, the focal point r5 of the paraboloidal mirror), and the paraboloidal mirror 71 in the listed sequence, and is emitted from the paraboloidal mirror 71 while being parallel to the symmetrical axis z1. For this reason, light fluxes are telecentric on both of a correction mirror system 71 side and a correction mirror system 72 side. An image plane is inclined by the reflection of the correction mirror systems 71 and 72 (refer to the intermediate image Ic2 in Fig. 2). The image plane is inclined in such a way as to cancel out the inclination of an image plane which occurs due to the first mirror 50 and the second mirror 60. As illustrated in Fig. 2, in a case where the inclination occurring due to the first mirror 50 and the second mirror 60 is non-linear, the correction mirror systems 71 and 72 desirably cancel out at least non-linear components of the inclination.

The first mirror 50 of the first example is a convex mirror, the convex surface of which faces the paraboloidal mirror 71 and the second mirror 60. That is, the first mirror 50 has negative power. The first mirror 50 is disposed eccentric (off-axis) with respect to the paraboloidal mirror 71. That is, the symmetrical axis z2, which is the symmetrical axis (that is, the object axis of a paraboloidal surface) of the mirror surface of the first mirror 50, is parallel to the symmetrical axis z1 of the paraboloidal mirror 71 while being spaced from the symmetrical axis z1. For this reason, the first mirror 50 is irradiated with beams from the paraboloidal mirror 71 which are parallel to the symmetrical axis z2. The first mirror 50 is disposed such that the focal point of the first mirror 50 coincides with the focal point r1 of the two focal points r1 and r2 of the second mirror 60 which is a spheroidal mirror. For this reason, in a case where a position on the first mirror 50 is irradiated with light from the paraboloidal mirror 71, the light is reflected along a straight line through which the irradiation position is connected to the focal point r1. That is, light from the first mirror 50 toward the second mirror 60 turns around the focal point r1 of the second mirror 60, which is a spheroidal mirror, in correspondence with the driving of the optical scanner 15 (or the optical scanner 27). In other words, a pivot point of light travelling toward the second mirror 60 through the optical scanner 15 (or the optical scanner 27) is formed at the focal point r1 by the first mirror 50. In the first example, since light is reflected by the mirror surface of the first mirror 50, the swing angle of light from the first pivot point r1 toward the second mirror 60 is further increased compared to the swing angle of light incident to the first mirror 50 from the scanning optical system 1. For this reason, in the first example, the swing angle of light incident to the first mirror 50 is further decreased compared to that of light incident to the second mirror 60. As an example, as illustrated in Fig. 1, telecentric light can be incident. In this case, it is considered that the swing angle is equal to zero.

Due to general characteristics of a spheroidal mirror, that is, the second mirror 60, light, which passes through the focal point r1 and is reflected by the mirror surface of the spheroidal mirror, is guided to the focal point r2. For this reason, a pivot point (the second pivot point) of light reflected by the second mirror 60 is formed at the focal point r2 (that is, the position of the anterior ocular segment of the subject's eye E). The swing angle of light around the pivot point (the focal point r2) is determined by a swing angle with respect to the focal point r1 and the shape of the mirror surface of the second mirror 60. The second mirror 60 may have a shape that further increases the swing angle with respect to the focal point r2 compared to the swing angle with respect to the focal point r1. The present invention is not necessarily limited to that configuration.

As such, in the first example, telecentric (swing angle = 0) light is incident to the objective optical system 2 (more specifically, the paraboloidal mirror 71) from the scanning optical system 1, and thus, it is possible to capture a wide-range image of the fundus Er. In the first example, the optical path coupling member 40 (the dichroic mirror 40), which couples the respective optical paths of the SLO optical system 10 and the OCT optical system 20 together, is disposed at a location in which the SLO optical system 10 and the OCT optical system 20 are telecentric. As a result, it is possible to obtain a good front image and a good tomographic image at a wide image angle.

Since the second mirror 60 is a spheroidal mirror, the asymmetric distortion (for example, trapezoidal distortion) of an image plane of fundus reflected light occurs due to the second mirror 60. In contrast, in the example, the first mirror 50 is disposed inclined with respect to the second mirror 60, and thus, the distortion of an image plane is prevented. That is, the first mirror 50 is disposed inclined with respect to a beam passing through the center of the optical paths between the first mirror 50 and the second mirror 60. The amount of correction of distortion of the image plane is changed in correspondence with the amount of inclination of the first mirror 50. The amount of inclination of the first mirror 50 may be set such that the remaining distortion of the image plane is axis-symmetric.

### <Control System>

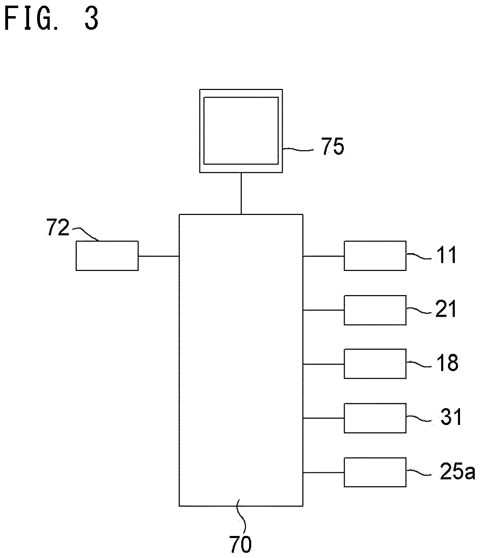

Hereinafter, a control system of the imaging device 100 will be described with reference to Fig. 3. A control unit 70 is a processor (for example, a CPU) that controls the entirety of the imaging device 100.

In the first example, the control unit 70 is electrically connected to a memory 72, a monitor 75 and the like. The control unit 70 is electrically connected to the light sources 11 and 21, the optical scanners 15 and 27, the detectors 18 and 31, the drive mechanism 25a, and the like.

The memory 72 stores various control programs and fixed data. The memory 72 may store images captures by the imaging device 100, temporary data, and the like.

In the embodiment, the control unit 70 also serves as an image processing unit. For example, received light signals from the detectors 18 and 31 are input to the control unit 70. The control unit 70 forms a front image of the fundus Er based on the signals from the detector 18. The control unit 70 forms a tomographic image of the fundus Er based on the signals from the detector 31. The control unit 70 may acquire both of a front image and a tomographic image by concurrently and independently driving light from the light source 11 and light from the light source 21 via the optical scanners 15 and 27. The front image and the tomographic image concurrently obtained may be concurrently displayed on the monitor 75 in the form of a moving image. In the first example, the optical scanner 15 of the SLO optical system 10 is provided independently from the optical scanner 27 of the OCT optical system 20, and thus, the control unit 70 may acquire a front image and a tomographic image at different frame rates.

### <Second Example>

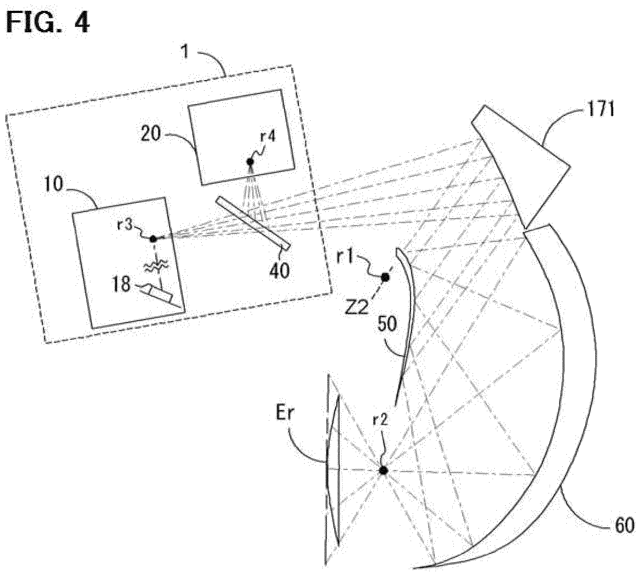

Hereinafter, a second example will be described with reference to Fig. 4. The same reference signs as those in the first example will be assigned to the same configuration elements of the second example as those in the first example, and description thereof will be omitted.

A difference between the second example and the first example is a portion of the scanning optical system 1 and the objective optical system 2. In the first example, the SLO optical system 10 and the OCT optical system 20 are telecentric with respect to an object side. In contrast, in the second example, light incident to the objective optical system 2 from the scanning optical system 1 (more specifically, the SLO optical system 10 and the OCT optical system 20) turns around a pivot point at a finite distance from the objective optical system 2. In the second example, for descriptive purposes, light from the SLO optical system 10 is assumed to turn around the pivot point r3 when incident to the objective optical system 2, and light from the OCT optical system 20 is assumed to turn around the pivot point r4 when incident to the objective optical system 2.

The objective optical system 2 of the second example includes a paraboloidal mirror 171 between the first mirror 50 and the optical scanners 15 and 27. The paraboloidal mirror 171 is a concave mirror which is disposed such that the focal point of the concave mirror coincides with the pivot points r3 and r4. For this reason, light fluxes are telecentric on an object side of the paraboloidal mirror 171. In the second example, the paraboloidal mirror 171 is disposed such that the object axis (that is, the symmetrical axis of a paraboloidal surface) (not illustrated) of a mirror surface of the paraboloidal mirror 171 is parallel to the symmetrical axis z2 of the mirror surface of the first mirror 50. For this reason, similar to the first example, the first mirror 50 is irradiated with beams from the optical scanner 15 or 27 side (that is, the paraboloidal mirror 171) which are parallel to the symmetrical axis z2. As a result, light from the first mirror 50 toward the second mirror 60 turns around the first pivot point r1 which is positioned where the focal point of the first mirror 50 overlaps with the focal point of the second mirror 60. Light is reflected by the second mirror 60, and thus, the light turns around the focal point r2 of the spheroidal mirror. Also, in the second example, as such, it is possible to decrease the swing angle of light incident to the objective optical system 2 (more specifically, the paraboloidal mirror 171) from the scanning optical system 1, and to satisfactorily scan a wide range on the fundus Er with light. As a result, it is possible to prevent partial deterioration of image quality of an image of the fundus which is caused by the incidence angle dependence of the optical path coupling member 40 (the dichroic mirror 40).

In a case where an image of the fundus is captured at a desired image angle, it is possible to decrease the swing angle of light in the optical scanners 15 and 27 by the extent of an increase in the focal distance of the paraboloidal mirror 171. For this reason, the paraboloidal mirror 171 having a longer focal distance within an allowable device size range is preferably adopted. As a result, it is considered that it is possible to more effectively reduce a problem of incidence angle dependence of the optical path coupling member 40.

The inventors of this application have confirmed that in a case where the optical system of the first example and the optical system of the second example are designed to conditions, better imaging performance is obtained in the second example than that in the first example.

The paraboloidal mirror 171 provided instead of the correction mirror systems 71 and 72 of the first example does not correct the inclination of an image plane. In contrast, in the second example, in order to prevent the inclination of an image plane in at least the SLO optical system 10, at least the detector 18 may be provided in such a way that the detector 18 is disposed inclined with respect to the optical axis of the light receiving optical system 10a. Furthermore, either the collimating lens 12 or the columnar lens 13 of the SLO optical system 10 may be disposed inclined with respect to the optical axis thereof. In the second example, the amount of inclination of the detector 18 is adjusted such that a Scheimpflug relationship is established between the fundus Er and the objective optical system 2. As a result, it is possible to prevent deterioration of image quality caused by the inclination of an image plane (that is, due to focus changing according to scan position).

### <Third Example>

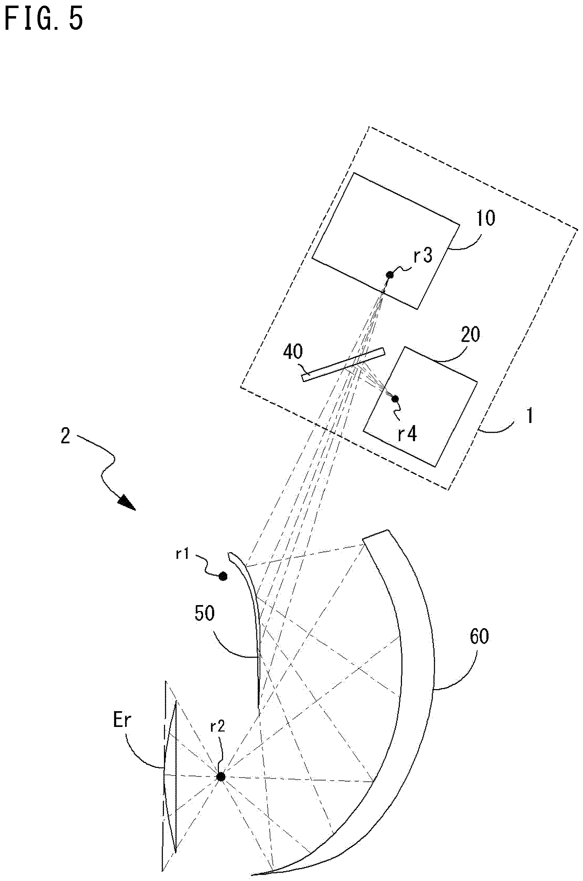

Hereinafter, a third example will be described with reference to Fig. 5. The same reference signs as those in the first example will be assigned to the same configuration elements of the third example as those in the first example, and description thereof will be omitted. A main difference between the third example and the first and second examples is the shape of the first mirror 50. In the third example, a mirror system is not provided between the first mirror 50 and the scanning optical system 1. In the third example, the scanning optical system 1 and the objective optical system 2 are partially different from those of the first example. In the first example, the SLO optical system 10 and the OCT optical system 20 are telecentric with respect to the object side. In contrast, in the third example, similar to the second example, light incident to the objective optical system 2 from the scanning optical system 1 (more specifically, the SLO optical system 10 and the OCT optical system 20) turns around a pivot point at a finite distance from the objective optical system 2.

In the third example, the first mirror 50 is one hyperboloidal mirror (one of a pair of hyperboloidal surfaces). In the third example, a hyperboloidal mirror is an aspherical mirror that contribute to angle widening. Similar to the first example, the second mirror 60 may be a spheroidal mirror. The hyperboloidal mirror, that is, the first mirror 50 is disposed such that a virtual image side focal point (focal point on a convex surface) coincides with the pivot points r3 and r4. The first mirror 50 is disposed such that a real image side focal point (focal point on a concave surface side) coincides with one focal point of the second mirror 60. That is, the first mirror 50 is irradiated with light emitted from the virtual image side (hyperboloidal surface side which pairs with the first mirror 50) focal point. As a result, due to general characteristics of the hyperboloidal mirror, light reflected by the first mirror 50 turns around the rear image side focal point r1 of the first mirror 50 in correspondence with the driving of the optical scanner 15 (or the optical scanner 27). Since the focal point r1 is a focal point of the second mirror 60 which is a spheroidal mirror, the pivot point of light reflected by the first mirror 50 is formed at the focal point r1 of the spheroidal mirror due to the disposition of the first mirror 50. In the third example, since light is reflected by the mirror surface of the first mirror 50, the swing angle of light from the first pivot point r1 toward the second mirror 60 is further increased compared to the swing angle of light incident to the first mirror 50 from the scanning optical system 1. Light reflected by the second mirror 60 turns around a pivot point, that is, the focal point r2 of the second mirror 60. Also, in the third example, as such, it is possible to decrease the swing angle of light incident to the objective optical system 2 (more specifically, the first mirror 50) from the scanning optical system 1, and to satisfactorily scan a wide range on the fundus Er with light.

The first mirror 50 of the third example is a hyperboloidal mirror (eccentricity > 1). The mirror shape of the first mirror 50 becomes more similar to a paraboloidal shape illustrated in the first example as the eccentricity of a hyperboloidal surface approaches one. For this reason, as the eccentricity of the hyperboloidal surface approaches one, the virtual image side focal point (focal point on the convex surface side) becomes more distant from the mirror surface, and approaches infinity. For this reason, in a case where an image of the fundus is captured at a desired image angle, it is possible to further decrease the swing angle of light in the optical scanners 15 and 27 as the eccentricity of the first mirror 50 approaches one. For this reason, a hyperboloidal mirror having eccentricity closer to one within an allowable device size range is preferably adopted as the first mirror 50. As a result, it is considered that it is possible to more effectively reduce a problem of incidence angle dependence of the optical path coupling member 40.

As illustrated in Fig. 5, the first mirror 50 may be disposed inclined with respect to the second mirror 60. In order to correct the inclination of an image plane occurring in this case, the detector 18 and the like of the SLO optical system 10 may be disposed inclined with respect to the optical axis.

### <Operation of Device When Acquiring Tomographic Image>

The objective optical system 2 of the examples is an objective mirror system that forms a pivot point (the pivot point r2 in the examples), which turns in correspondence with the operation of the optical scanner 27, in the anterior ocular segment (for example, the position of the pupil) of the subject's eye E via mirror systems (the first mirror 50, the second mirror 60, and the like) which are disposed between the subject's eye E and the optical scanner 27. In the objective optical system 2 of the examples, the second mirror 60 immediately before the subject's eye E is a spheroidal mirror, and the pivot point r2 is formed at one of the two focal points r1 and r2 of the spheroidal mirror. The optical path length of light, which is incident to the mirror surface from one of the two focal points of the spheroidal mirror and is guided to the other focal point, is always the same between the two focal points. In contrast, in the examples, mirrors such as the first mirror 50 are disposed between the optical scanner 27 and the second mirror 60, and thus, the distance of measurement light from the optical scanner 27 to the second pivot point r2 differs according to scan position of the optical scanner 27.

In the examples, the optical path length of measurement light from the second pivot point (position of the pupil) r2 to a front surface of the fundus Er also differs between scan positions. That is, due to curvature of the fundus, the distance of measurement light from the second pivot point r2 to the fundus Er differs according to scan position of the optical scanner 27.

It is considered that the distance of measurement light from the optical scanner 27 to the subject's eye E differs between scan positions of the optical scanner 27. That is, it is considered that a change in an optical path length difference between measurement light and reference light occurs due to the distance of measurement light from the optical scanner 27 to the subject's eye E at each scan position of the optical scanner 27.

It is considered that in a case where depth profiles (OCT data) are obtained in this state based on signal from the detector 31, a depth position in an area, in which the depth profiles (OCT data) of the subject's eye E are obtained, differs between scan positions of the optical scanner 27. It is considered that since an optical path length difference depending on scan position, a deviation between a range in which the sensitivity of the detector 31 is high and a range in which interference between measurement light and reference light occurs is relatively large.

In contrast, the control unit 70 corrects a change in an optical path length difference between measurement light and reference light caused by the distance of measurement light from the optical scanner 27 to the subject's eye E at each scan position of the optical scanner 27.

A change in an optical path length difference between measurement light and reference light may be corrected via processing of data (via processing of OCT data). The control unit 70 may correct information regarding position in the depth direction for OCT data when the control unit 70 acquires the OCT data based on signals from the detector 31.

When forming two-dimensional OCT data by aligning multiple items of OCT data, the control unit 70 may correct a relative depth position between the OCT data for scan positions.

It is possible to obtain good OCT data (or two-dimensional OCT data) as the result of performing such a process.

A change in an optical path length difference between reference light and measurement light may be optically corrected. For example, correction may be performed by controlling the driving of the optical path length adjustment mechanism (drive mechanism) 25a in correspondence with the scan position of the optical scanner 27. As described above, the drive mechanism 25a may adjust an optical path length difference between measurement light and reference light by displacing an optical member (for example, a mirror) disposed on optical paths of the reference light (or optical paths of the measurement light). For example, in the examples, the drive mechanism 25a changes the optical path length of reference light in correspondence with a change in the optical path length of measurement light caused by the operation of the optical scanner 27. As a result, signals based on interference between measurement light and reference light are likely to be detected in a range in which the sensitivity of the detector 31 is high, and it is possible to obtain good OCT data (or two-dimensional OCT data). In this case, preferably, it is possible to decrease the range of change in an optical path length difference such that, regardless of scan position, signals based on interference between measurement light and reference light are detected in a range in which the sensitivity of the detector 31 is relatively high. The driving of the drive mechanism 25a is not necessarily required to be controlled such that an optical path length difference between measurement light and reference light is the same (for example, zero) between scan positions.

The control unit 70 corrects a change in an optical path length difference between measurement light and reference light while taking into consideration at least a change in the distance (optical path length) of the measurement light from the optical scanner 27 to the second pivot point r2. The control unit 70 may perform correction while taking into consideration a change in the optical path length of measurement light between scan positions (that is, a change in the optical path length of measurement light from the second pivot point r2 to the fundus Er) caused by the curvature of the fundus. A relationship between the optical path length of measurement light from the optical scanner 27 to the second pivot point r2 and scan positions of the optical scanner 27 is determined by design of an optical system. A relationship between the optical path length of measurement light from the second pivot point r2 and the fundus Er and scan positions of the optical scanner 27 is also determined by design (mainly swing angle with respect to the second pivot point) of an optical system.

For example, a correction table, in which the amounts of correction (for example, the amounts of change of an optical path length, or the amounts of correction of information regarding position in the depth direction for OCT data) required to correct changes in an optical path length difference are mapped to scan positions of the optical scanner 27, may be prepared in the memory 72 in advance. The control unit 70 may correct a change in an optical path length difference using the correction table. The amounts of correction in the table are values determined in consideration of at least a change in the optical path length of measurement light from the optical scanner 27 to the second pivot point r2. The amounts of correction may be values determined in consideration of a change in an optical path length from the second pivot point r2 to the fundus Er. A relationship between an optical path length difference and the amount of correction (in other words, a relationship between scan position and the amount of correction) may be obtained in advance via simulation, calibration, and the like.

In the examples, the optical scanner 27 includes two optical scanners. That is, the optical scanner 27 includes a first optical scanner (for example, an X galvanometer scanner) that performs a horizontal scan of a target with measurement light, and a second scanner (for example, a Y galvanometer scanner) that performs a vertical scan of the target with measurement light in a direction intersecting a horizontal scan direction. In a case where the horizontal scan is performed in a depth direction of each drawing sheet, and the vertical scan is performed in a direction intersecting the depth direction of the drawing sheet, it is possible to adopt the objective optical system 2 that causes a change in the optical path length of measurement light (more specifically, a change in an optical path length from the optical scanner 27 to the second pivot point r2) in correspondence with only the positions of vertical scan. For example, as in the embodiment, it is possible to realize the objective optical system 2 by using a concave mirror and a convex mirror which have rotating curved surfaces of a spheroidal mirror and a rotating paraboloidal mirror.

The control unit 70 may acquire two-dimensional OCT data for multiple scan lines by perform a raster scan of the objective optical system 2 with measurement light. That is, the control unit 70 acquires multiple items of two-dimensional OCT data, which is acquired via performing a horizontal scan, for different scan lines in a vertical scan direction by controlling the driving of the optical scanner 27. The driving of the drive mechanism 25a may be controlled in correspondence with the scan position of the second scanner. That is, a change in an optical path length difference between reference light and measurement light may be corrected by driving the drive mechanism 2 in correspondence with the scan position of the second scanner.

In this case, a change in an optical path length difference between measurement light and reference light may occur due to vertical scan, and in contrast, the speed of vertical scan is slower than that of horizontal scan. For this reason, it is possible to prevent a change in an optical path length difference over time. Accordingly, it is possible to drive the drive mechanism 25a such that a change in an optical path length difference between reference light and measurement light is more reliably corrected. As a result, it is possible to acquire multiple items of good two-dimensional OCT data.

The description has been given based on the embodiment; however, this disclosure is not limited to the embodiment, and change may be made in various forms.

In the first to third examples, the second mirror 60 of the objective optical system 2 is a spheroidal mirror, and the first mirror 50 used in combination therewith is a paraboloidal mirror or a hyperboloidal mirror. In other words, in the first to third examples, the first mirror 50 has a curve surface formed along a trajectory that is obtained by rotating a quadratic curve having eccentricity ≥ 1 around a symmetrical axis. As described above, the curved surface shape of each of the first mirror 50 and the second mirror 60 may be suitably selected from various quadric surfaces or various aspherical surfaces.

In the scanning optical system 1 of the embodiment, two imaging optical systems (the SLO optical system 10 and the OCT optical system 20 in the embodiment) respectively include optical scanners, and respective optical paths of the two imaging optical systems are coupled together by the optical path coupling member 40. This disclosure is not necessarily limited to that configuration. Alternatively, in the scanning optical system 1, optical paths of an imaging optical system and optical paths of an irradiation optical system which emits therapy light or stimulating light may be coupled together by the optical path coupling member 40. The imaging optical system referred to here may scan the fundus with light from a first light source for capturing an image by driving a first optical scanner, and may include a detector that receives fundus reflected light of light from the first light source. In this case, either the SLO optical system 10 or the OCT optical system 20 of the embodiment may be used as the imaging optical system. In contrast, light with which the subject's eye E is irradiated with the irradiation optical system may be therapy laser beams which coagulate the fundus with light. The light may be stimulating light for visual field test. Needless to say, the therapy light or the stimulating light are not limited to those purposes. The irradiation optical system may include at least a second optical scanner that determines the irradiation position of light on the fundus by deflecting light from a second light source emitting therapy light or stimulating light. The irradiation optical system may be replaced with either the SLO optical system 10 or the OCT optical system 20 of the embodiment. The second optical scanner may be replaced with either the optical scanner 15 or the optical scanner 27 of the embodiment, and the replacement one may be disposed.

## Claims

1. A fundus imaging device (100) for forming an image of a fundus of a subject's eye, the fundus imaging device (100) comprising:
a scanning optical system (1) that includes a first and a second optical scanner (15, 27) respectively configured to change a travelling direction of light emitted from a first and a second light source (11, 21) to scan the fundus with the light to form the image of the fundus based on the light reflected from the fundus; and
an objective optical system (2) that is disposed between the optical scanners (15, 27) and the subject's eye (E), and is configured to guide the light from the optical scanners (15,27) to the fundus,
wherein the objective optical system (2) includes:
a first mirror (50) configured to reflect the light from the optical scanners (15, 27), and form a first pivot point (r1) around which the light turns in correspondence with the operation of the optical scanners (15, 27), wherein the first mirror (50) is an aspherical mirror, the mirror surface of which is a quadric surface formed along a trajectory obtained by rotating a quadratic curve around a symmetrical axis, and
a second mirror (60) configured to reflect the light reflected by the first mirror (50), and form a second pivot point (r2) around which the light emitted to the subject's eye (E) turns, wherein a swing angle of the light incident to the first mirror (50) is smaller compared to a swing angle of the light with respect to the second pivot point (r2), and
wherein the scanning optical system (1) includes:
an SLO optical system (10) that includes the first optical scanner (15) configured to scan light from the first light source (11), the SLO optical system (10) being configured to obtain a front image of the light reflected from the fundus,
an OCT optical system (20) that includes the second optical scanner (27) configured to scan measurement light from the second light source (21), and is configured to obtain a tomographic image of the subject's eye using optical interference, and
an optical path coupling member (40) that is disposed between the first optical scanner (15) and the first mirror (50) and between the second optical scanner (27) and the first mirror (50), and is configured to couple optical paths of the SLO optical system (10) and optical paths of the OCT optical system (20) together, **characterized in that**
the second optical scanner is formed of two galvanometer mirrors, and configured to scan the fundus (Er) with the measurement light in a two-dimensional (XY) direction.

2. The fundus imaging device according to claim 1, wherein the first mirror (50) turns light, which is incident to the second mirror (60) from the first pivot point (r1), around the first pivot point (r1) at a larger swing angle compared to a swing angle of the light incident to the first mirror (50) from the scanning optical system (1).

3. The fundus imaging device according to claim 1 or 2, wherein
the first mirror (50) forms the first pivot point (r1) at a focal point of the first mirror (50), and
the second mirror (60) has two focal points, and forms the second pivot point (r2) at one of the focal points and the first pivot point (r1) is formed at the other focal point.

4. The fundus imaging device according to claim 1, wherein the aspherical mirror is at least any one of a paraboloidal mirror, a hyperboloidal mirror, and an ellipsoidal mirror.

5. The fundus imaging device according to claim 1, wherein
the first mirror (50) is a paraboloidal mirror or a hyperboloidal mirror, which has a convex surface as a reflective surface, and
the second mirror (60) is an ellipsoidal mirror that has a concave surface as a reflective surface.

6. The fundus imaging device according to any one of claims 1 to 4, further comprising:
an optical member configured to correct an inclination of an image plane which occurs due to the light being reflected by the first mirror and the second mirror.

7. The fundus imaging device according to claim 6, wherein the objective optical system (2) includes a correction mirror system between the first mirror (50) and the optical scanner (15, 27), and the correction mirror system is configured to correct the inclination of the image plane.

8. The fundus imaging device according to claim 7, wherein
the first mirror (50) is a paraboloidal mirror that has a convex surface as a reflective surface, and
the correction mirror system (71, 72) includes a second paraboloidal mirror configured to guides the light, which is scanned by the optical scanner, to the first mirror (50) in a telecentric manner.

9. The fundus imaging device according to claim 6, wherein
the scanning optical system (1) includes a line sensor or an area sensor as a detector (31) configured to receive the light reflected from the fundus, and a line-scan SLO optical system (10) configured to scan the fundus with line-shaped light fluxes via the optical scanner, and
the optical member is the detector disposed inclined with respect to the optical axis thereof.

10. The fundus imaging device according to claim 9, wherein the detector is inclinedly disposed such that a Scheimpflug relationship is established between the fundus and the objective optical system.

11. The fundus imaging device according to any one of claims 1 to 10, wherein the first mirror (50) is configured to correct asymmetric distortion of an image plane which occurs due to the second mirror (60).

12. The fundus imaging device according to claim 1, wherein the SLO optical system (10) and the OCT optical system (20) are telecentric with respect to an object side.

## Patentansprüche

1. Fundusabbildungsvorrichtung (100) zum Formen eines Bildes eines Fundus eines Subjektauges, wobei die Fundusabbildungsvorrichtung (100) aufweist:
ein scannendes optisches System (1), das einen ersten und einen zweiten optischen Scanner (15, 27) aufweist, die jeweils konfiguriert sind, um eine Ausbreitungsrichtung von Licht zu ändern, das von einer ersten und einer zweiten Lichtquelle (11, 21) emittiert ist, um den Fundus mit dem Licht zu scannen, um das Bild des Fundus basierend auf dem von dem Fundus reflektierten Licht zu formen; und
ein objektives optisches System (2), das zwischen den optischen Scannern (15, 27) und dem Subjektauge (E) angeordnet ist und konfiguriert ist, um das Licht von den optischen Scannern (15, 27) zu dem Fundus zu führen,
wobei das objektive optische System (2) aufweist:
einen ersten Spiegel (50), der konfiguriert ist, um das Licht von den optischen Scannern (15, 27) zu reflektieren und einen ersten Drehpunkt (r1) zu formen, um den sich das Licht in Übereinstimmung mit der Betätigung der optischen Scanner (15, 27) dreht, wobei der erste Spiegel (50) ein asphärischer Spiegel ist, dessen Spiegeloberfläche eine quadratische Oberfläche ist, die entlang einer Trajektorie geformt ist, die durch Drehen einer quadratischen Kurve um eine symmetrische Achse erhalten ist, und
einen zweiten Spiegel (60), der konfiguriert ist, um das durch den ersten Spiegel (50) reflektierte Licht zu reflektieren und einen zweiten Drehpunkt (r2) zu formen, um den sich das zum Subjektauge (E) emittierte Licht dreht, wobei ein Schwenkwinkel des auf den ersten Spiegel (50) einfallenden Lichts kleiner ist als ein Schwenkwinkel des Lichts in Bezug auf den zweiten Drehpunkt (r2), und
wobei das scannende optische System (1) aufweist:
ein optisches SLO-System (10), das den ersten optischen Scanner (15) aufweist, der konfiguriert ist, um Licht von der ersten Lichtquelle (11) zu scannen, wobei das optische SLO-System (10) konfiguriert ist, um ein Frontbild des von dem Fundus reflektierten Lichts zu erhalten,
ein optisches OCT-System (20), das den zweiten optischen Scanner (27) aufweist, der konfiguriert ist, um Messlicht von der zweiten Lichtquelle (21) zu scannen, und der konfiguriert ist, um ein tomographisches Bild des Subjektauges unter Verwendung von optischer Interferenz zu erhalten, und
ein optisches Pfadkopplungselement (40), das zwischen dem ersten optischen Scanner (15) und dem ersten Spiegel (50) und zwischen dem zweiten optischen Scanner (27) und dem ersten Spiegel (50) angeordnet ist und konfiguriert ist, um optische Pfade des optischen SLO-Systems (10) und optische Pfade des optischen OCT-Systems (20) miteinander zu koppeln,
**dadurch gekennzeichnet, dass** der zweite optische Scanner aus zwei Galvanometerspiegeln geformt ist und konfiguriert ist, um den Fundus (Er) mit dem Messlicht in einer zweidimensionalen (XY) Richtung zu scannen.

2. Fundusabbildungsvorrichtung nach Anspruch 1, wobei der erste Spiegel (50) das vom ersten Drehpunkt (r1) auf den zweiten Spiegel (60) einfallende Licht um den ersten Drehpunkt (r1) mit einem größeren Schwenkwinkel im Vergleich zu einem Schwenkwinkel des vom scannenden optischen System (1) auf den ersten Spiegel (50) einfallenden Lichts dreht.

3. Fundusabbildungsvorrichtung nach Anspruch 1 oder 2, wobei
der erste Spiegel (50) den ersten Drehpunkt (r1) an einem Fokuspunkt des ersten Spiegels (50) formt, und
der zweite Spiegel (60) zwei Fokuspunkte aufweist und den zweiten Drehpunkt (r2) an einem der Fokuspunkte formt und der erste Drehpunkt (r1) an dem anderen Fokuspunkt geformt ist.

4. Fundusabbildungsvorrichtung nach Anspruch 1, wobei der asphärische Spiegel mindestens einer von einem Parabolspiegel, einem Hyperboloidspiegel und einem Ellipsoidspiegel ist.

5. Fundusabbildungsvorrichtung nach Anspruch 1, wobei
der erste Spiegel (50) ein Paraboloidspiegel oder ein Hyperboloidspiegel ist, der eine konvexe Oberfläche als eine reflektierende Oberfläche aufweist, und
der zweite Spiegel (60) ein ellipsoidischer Spiegel ist, der eine konkave Oberfläche als eine reflektierende Oberfläche aufweist.

6. Fundusabbildungsvorrichtung nach einem der Ansprüche 1 bis 4, ferner umfassend:
ein optisches Element, das konfiguriert ist, um eine Neigung einer Bildebene zu korrigieren, die dadurch entsteht, dass das Licht durch den ersten Spiegel und den zweiten Spiegel reflektiert ist.

7. Fundusabbildungsvorrichtung nach Anspruch 6, wobei das objektive optische System (2) ein Korrekturspiegelsystem zwischen dem ersten Spiegel (50) und dem optischen Scanner (15, 27) aufweist und das Korrekturspiegelsystem konfiguriert ist, um die Neigung der Bildebene zu korrigieren.

8. Fundusabbildungsvorrichtung nach Anspruch 7, wobei
der erste Spiegel (50) ein Paraboloidspiegel ist, der eine konvexe Oberfläche als eine reflektierende Oberfläche aufweist, und
das Korrekturspiegelsystem (71, 72) einen zweiten Parabolspiegel aufweist, der konfiguriert ist, um das Licht, das durch den optischen Scanner gescannt ist, in einer telezentrischen Weise zu dem ersten Spiegel (50) zu führen.

9. Fundusabbildungsvorrichtung nach Anspruch 6, wobei
das scannende optische System (1) einen Zeilensensor oder einen Flächensensor als einen Detektor (31), der zum Empfangen des von dem Fundus reflektierten Lichts konfiguriert ist, und ein zeilen-scannendes optisches SLO-System (10), das zum Scannen des Fundus mit zeilenförmigen Lichtflüssen mittels des optischen Scanners konfiguriert ist, aufweist, und
das optische Element der Detektor ist, der in Bezug auf seine optische Achse geneigt angeordnet ist.

10. Fundusabbildungsvorrichtung nach Anspruch 9, wobei der Detektor geneigt angeordnet ist, sodass eine Scheimpflug-Beziehung zwischen dem Fundus und dem objektiven optischen System entsteht.

11. Fundusabbildungsvorrichtung nach einem der Ansprüche 1 bis 10, wobei der erste Spiegel (50) konfiguriert ist, um asymmetrische Verzerrung einer Bildebene zu korrigieren, die aufgrund des zweiten Spiegels (60) entsteht.

12. Fundusabbildungsvorrichtung nach Anspruch 1, wobei das optische SLO-System (10) und das optische OCT-System (20) in Bezug auf eine Objektseite telezentrisch sind.

## Revendications

1. Dispositif d'imagerie de fond d'oeil (100) pour former une image d'un fond d'oeil de l'œil d'un sujet, le dispositif d'imagerie de fond d'oeil (100) comprenant :
un système optique de balayage (1) qui comprend un premier et un deuxième dispositif de balayage optique (15, 27) configurés respectivement pour changer une direction de déplacement de la lumière émise par une première et une deuxième source de lumière (11, 21) pour balayer le fond d'oeil avec la lumière afin de former l'image du fond d'oeil sur la base de la lumière réfléchie par le fond d'oeil ; et
un système optique objectif (2) qui est disposé entre les dispositifs de balayage optiques (15, 27) et l'œil du sujet (E), et est configuré pour guider la lumière des dispositifs de balayage optiques (15, 27) vers le fond d'oeil,
dans lequel le système optique objectif (2) comprend :
un premier miroir (50) configuré pour réfléchir la lumière provenant des dispositifs de balayage optiques (15, 27) et former un premier point de pivotement (r1) autour duquel la lumière tourne en correspondance avec le fonctionnement des dispositifs de balayage optiques (15, 27), dans lequel le premier miroir (50) est un miroir asphérique, dont la surface de miroir est une surface quadrique formée le long d'une trajectoire obtenue en faisant tourner une courbe quadratique autour d'un axe de symétrie, et
un deuxième miroir (60) configuré pour réfléchir la lumière réfléchie par le premier miroir (50), et former un deuxième point de pivotement (r2) autour duquel tourne la lumière émise vers l'œil (E) du sujet, dans lequel un angle d'oscillation de la lumière incident sur le premier miroir (50) est plus petit que l'angle d'oscillation de la lumière par rapport au deuxième point de pivotement (r2), et
dans lequel le système optique de balayage (1) comprend :
un système optique SLO (10) qui comprend le premier dispositif de balayage optique (15) configuré pour balayer une lumière provenant de la première source de lumière (11), le système optique SLO (10) étant configuré pour obtenir une image frontale de la lumière réfléchie par le fond d'oeil,
un système optique OCT (20) qui comprend le deuxième dispositif de balayage optique (27) configuré pour balayer une lumière de mesure provenant de la deuxième source de lumière (21), et est configuré pour obtenir une image tomographique de l'œil du sujet au moyen d'une interférence optique, et
un élément de couplage de chemin optique (40) qui est disposé entre le premier dispositif de balayage optique (15) et le premier miroir (50) et entre le deuxième dispositif de balayage optique (27) et le premier miroir (50), et est configuré pour coupler des chemins optiques du système optique SLO (10) et des chemins optiques du système optique OCT (20) ensemble, **caractérisé en ce que**
le deuxième dispositif de balayage optique est formé de deux miroirs de galvanomètre, et configuré pour balayer le fond d'oeil (Er) avec la lumière de mesure dans une direction bidimensionnelle (XY).

2. Dispositif d'imagerie de fond d'oeil selon la revendication 1, dans lequel le premier miroir (50) fait tourner la lumière, qui est incidente sur le deuxième miroir (60) depuis le premier point de pivotement (r1), autour du premier point de pivotement (r1) à un angle d'oscillation plus grand qu'un angle d'oscillation de la lumière incidente sur le premier miroir (50) depuis le système optique de balayage (1).

3. Dispositif d'imagerie de fond d'oeil selon la revendication 1 ou 2, dans lequel
le premier miroir (50) forme le premier point de pivotement (r1) à un point focal du premier miroir (50), et
le deuxième miroir (60) a deux points focaux et forme le deuxième point de pivotement (r2) à l'un des points focaux et le premier point de pivotement (r1) est formé à l'autre point focal.

4. Dispositif d'imagerie de fond d'oeil selon la revendication 1, dans lequel le miroir asphérique est au moins l'un quelconque parmi un miroir paraboloïdal, un miroir hyperboloïde et un miroir ellipsoïdal.

5. Dispositif d'imagerie de fond d'oeil selon la revendication 1, dans lequel
le premier miroir (50) est un miroir paraboloïdal ou un miroir hyperboloïde, qui a une surface convexe en tant que surface réfléchissante, et
le deuxième miroir (60) est un miroir ellipsoïdal qui a une surface concave en tant que surface réfléchissante.

6. Dispositif d'imagerie de fond d'oeil selon l'une quelconque des revendications 1 à 4, comprenant en outre :
un élément optique configuré pour corriger une inclinaison d'un plan d'image due à la réflexion de la lumière par le premier miroir et le deuxième miroir.

7. Dispositif d'imagerie de fond d'oeil selon la revendication 6, dans lequel le système optique objectif (2) comprend un système de miroir de correction entre le premier miroir (50) et le dispositif de balayage optique (15, 27), et le système de miroir de correction est configuré pour corriger l'inclinaison du plan image.

8. Dispositif d'imagerie de fond d'oeil selon la revendication 7, dans lequel
le premier miroir (50) est un miroir paraboloïdal qui a une surface convexe en tant que surface réfléchissante, et
le système de miroir de correction (71, 72) comprend un deuxième miroir paraboloïdal configuré pour guider la lumière, qui est balayée par le dispositif de balayage optique, vers le premier miroir (50) d'une manière télécentrique.

9. Dispositif d'imagerie de fond d'oeil selon la revendication 6, dans lequel
le système optique de balayage (1) comprend un capteur de ligne ou un capteur de surface en tant que détecteur (31) configuré pour recevoir la lumière réfléchie par le fond d'oeil, et un système optique SLO à balayage linéaire (10) configuré pour balayer le fond d'oeil avec des flux lumineux en forme de ligne via le dispositif de balayage optique, et
l'élément optique est le détecteur disposé incliné par rapport à son axe optique.

10. Dispositif d'imagerie de fond d'oeil selon la revendication 9, dans lequel le détecteur est disposé de manière inclinée de sorte qu'une relation de Scheimpflug soit établie entre le fond d'oeil et le système optique objectif.

11. Dispositif d'imagerie de fond d'oeil selon l'une quelconque des revendications 1 à 10, dans lequel le premier miroir (50) est configuré pour corriger la distorsion asymétrique d'un plan d'image due au deuxième miroir (60).

12. Dispositif d'imagerie de fond d'oeil selon la revendication 1, dans lequel le système optique SLO (10) et le système optique OCT (20) sont télécentriques par rapport à un côté objet.
